# EUROPEAN PATENT SPECIFICATION

(11) **EP 3 454 293 B1**
(45) Date of publication and mention of the grant of the patent: **18.03.2020**
(21) Application number: 18192679.1
(22) Date of filing: 05.09.2018
(51) Int. Cl.: G06T 5/00, G06T 5/50, G06T 7/11, G06T 7/174, G06T 7/187, G06T 7/136

(54) **METHOD AND APPARATUS FOR ENHANCEMENT OF BRONCHIAL AIRWAYS REPRESENTATIONS USING VASCULAR MORPHOLOGY**
VERFAHREN UND VORRICHTUNG ZUR VERBESSERUNG DER DARSTELLUNG DER BRONCHIALEN ATEMWEGE UNTER VERWENDUNG VON VASKULÄRER MORPHOLOGIE
PROCÉDÉ ET APPAREIL D'AMÉLIORATION DE REPRÉSENTATIONS DES VOIES RESPIRATOIRES BRONCHIQUES UTILISANT LA MORPHOLOGIE VASCULAIRE

(30) Priority: 06.09.2017 US 201715696519
(43) Date of publication of application: 13.03.2019
(73) Proprietor: Biosense Webster (Israel) Ltd., 2066717 Yokneam (IL)
(72) Inventor: ZOABI, Akram, 2066717 Yokneam (IL); ZRIHEM, Yaniv Ben, 2066717 Yokneam (IL); DEKEL, Zvi, 2066717 Yokneam (IL)
(74) Representative: Small, Gary James

(56) References cited:
- US-A1- 2007 092 864
- US-A1- 2012 268 450
- US-A1- 2015 379 760

## Description

### SUMMARY

Methods and apparatus are provided for enhancement of bronchial airways representations using vascular morphology.

In one embodiment, a representation of the bronchial airways of a subject and a representation of the corresponding vascular network are initially obtained. In the bronchial airways representation, spatial coordinates are identified of termini of airways where portions of the airways are not included in the representation though they are in fact continuous airways in the subject's anatomy. A vessel portion is then identified in the vascular network representation that corresponds to an unrepresented airway portion between first and second identified termini of airways in the bronchial airways representation. With this data, an enhanced bronchial airways representation is produced that reflects an airway extending between the first and second termini. The enhanced bronchial airways representation can be displayed on a monitor of a medical apparatus to facilitate bronchoscopic procedures.

In the vascular network representation, locations that correspond to the identified termini of airways in the bronchial airways representation can be identified. When two identified locations in the vascular network representation are connected by a vascular path of a blood vessel represented in the vascular network representation, the identified termini can be selected as the first and second termini and used to enhance the bronchial airways representation, reflecting an airway extending therebetween.

The method can be performed so that the bronchial airways representation and the vascular network representation are obtained from a single computed tomography (CT) scan. The bronchial airways representation can be generated by setting a Hounsfield unit threshold for region growing segmentation of the CT scan for airway detection. The enhanced bronchial airways representation can be generated by setting a lower Hounsfield unit threshold for region growing segmentation of the CT scan around detected vessels both in terms of location and orientation of detected vessels. Alternatively, the bronchial airways representation and the vascular network representation can be obtained from separate CT scans. The CT scan used to obtain the vascular network representation can be enhanced via a contrast agent.

A display of the vascular network representation can be used by a physician or other professional to manually identify a vessel portion in the vascular network representation that corresponds to an unrepresented airway portion. Alternatively, the identifying of locations in the vascular network representation that correspond to the termini of airways in the bronchial airways can be determined by a processor.

An example apparatus for providing an enhanced representation of bronchial airways of a subject includes a memory configured to store an initial data representation of the bronchial airways of the subject. The data representation includes spatial coordinates of termini of airways where the airways continue in the subject, but continuations of the airways are not included in the initial bronchial airways representation. The memory is further configured to store a data representation of a vascular network corresponding to the bronchial airways of the subject. The apparatus also includes a processor configured to identify a vessel portion in the vascular network representation that corresponds to an unrepresented airway portion between first and second identified termini of airways in the bronchial airways representation. The processor is further configured to produce an enhanced bronchial airways representation that reflects an airway extending between the first and second termini.

To do this, the processor can be configured to identify locations in the vascular network representation that correspond to the identified termini of airways in the bronchial airways representation. When two identified locations in the vascular network representation are connected by a vascular path of a blood vessel represented in the vascular network representation, the processor can be configured to select the two identified termini as the first and second termini for which the enhanced bronchial airways representation reflects an airway extending therebetween. In this way, the apparatus can identify a vessel portion in the vascular network representation that corresponds to an unrepresented airway between first and second identified termini of airways in the bronchial airways representation.

The apparatus can include a display configured to display the enhanced bronchial airways representation. The display can be a monitor of a medical apparatus configured to assist navigation of a bronchoscope in a bronchoscopic procedure. The display can also be used to manually identify a vessel portion in the vascular network representation that corresponds to an unrepresented airway portion.

The memory can be configured to store the bronchial airways representation and the vascular network representation obtained from a single computed tomography scan. The processor can be configured to generate this bronchial airways representation by setting a Hounsfield unit threshold for region growing segmentation of the CT scan for airway detection. The processor may generate the enhanced bronchial airways representation by setting a lower Hounsfield unit threshold for region growing segmentation of the CT scan around detected vessels both in terms of location and orientation of detected vessels. Alternatively, the memory can be configured to store the bronchial airways representation and the vascular network representation as obtained from separate computed tomography scans. The memory can be further configured to store the vascular network representation as obtained from a computed tomography scan enhanced via a contrast agent.

Other objects and advantages of the invention will be apparent to those of skill in the art from the following detailed description and accompanying drawings.

### BRIEF DESCRIPTION OF THE DRAWINGS

The present embodiments are illustrated by way of example, and not by way of limitation, in the figures of the accompanying drawings.
Fig. 1 is a schematic illustration of a respiratory bronchial tree.
Fig. 2 is an example schematic illustration of a computed tomography (CT) scan system for providing three dimensional representations of a subject's lungs made in accordance with the teachings of the present invention.
Fig. 3A is an illustration of an example of a segmentation representation of bronchial airways of a subject derived from a CT scan.
Fig. 3B is an enlarged portion of the illustration of the segmentation representation of bronchial airways of Fig. 3A.
Fig. 4A is an illustration of a portion of a vascular segmentation representation of blood vessels corresponding to the bronchial airways of Fig. 3A.
Fig. 4B is an enlarged portion of the illustration of the vascular segmentation representation of Fig. 4A showing vessels in the region corresponding to the several airway termini depicted in Fig. 3B.
Figs. 5A-5C are examples of portions of 2-D segments from which the example airways and vascular segmentation representations of Figs. 3B and 4B were derived. Fig. 5A reflects a partial segment along line A-A of Figs. 3B and 4B, Fig. 5B reflects a partial segment along line B-B of Figs. 3B and 4B, and Fig. 5C reflects a partial segment along line C-C of Figs. 3B and 4B.
Fig. 6 is an illustration of an example enhanced segmentation representation of bronchial airways of a subject in accordance with the teachings of the present invention.
Fig. 7 is a flow diagram of an example method of providing an enhanced representation of bronchial airways of a subject in accordance with the teachings of the present invention.
Fig. 8 is an illustration of an example medical apparatus used to perform diagnostic and other bronchoscopic procedures that incorporates enhanced bronchial airways representation production according to the teachings of the present invention.

### DETAILED DESCRIPTION OF THE EMBODIMENTS

Computed tomography (CT) scans are well known in the medical arts. CT scans are used not only for diagnostic purposes, but also to aid in the navigation of catheters and/or other probes for relatively non-invasive medical procedures. The CARTO™ mapping system produced by Biosense Webster Inc., of Diamond Bar, Calif., for which various details are described in U.S. Patent Application Publications 2009/0093806 and 2009/0138007 and U.S. Pat. Nos. 5,391,199, 5,443,489, 5,558,091, 5,983,126, 5,944,022, 6,172,499, and 6,177,792, 6,788,967, and 6,690,963, employs CT scan data to assist in the navigation of catheters and other probes in a subject's heart. A CT scan series of essentially two dimensional segments are analyzed to provide a three-dimensional segmentation representation of the heart for this purpose.

US2015379760A1 discloses a method for using an assembled three-dimensional image to construct a three-dimensional model for determining a path through a lumen network to a target. The three-dimensional model is automatically registered to an actual location of a probe by tracking and recording the positions of the probe and continually adjusting the registration between the model and a display of the probe position.
US20070092864A1 discloses treatment planning methods, devices and systems. One of the treatment planning methods includes displaying at least a portion of a set of lungs and one or more potential treatment regions; receiving a selection of a target treatment region from among the one or more potential treatment regions; and displaying one or more locations within the portion that are therapeutically linked to the target treatment region.

US20120268450A1 discloses a method including displaying a three-dimensional (3D) image of a lung, receiving a selection of an airway of the lung and displaying a two-dimensional (2D) cross-section image of the airway perpendicular to the airway's long axis. The display of the 2D cross-section image occurs almost immediately after the selection of the airway is received.

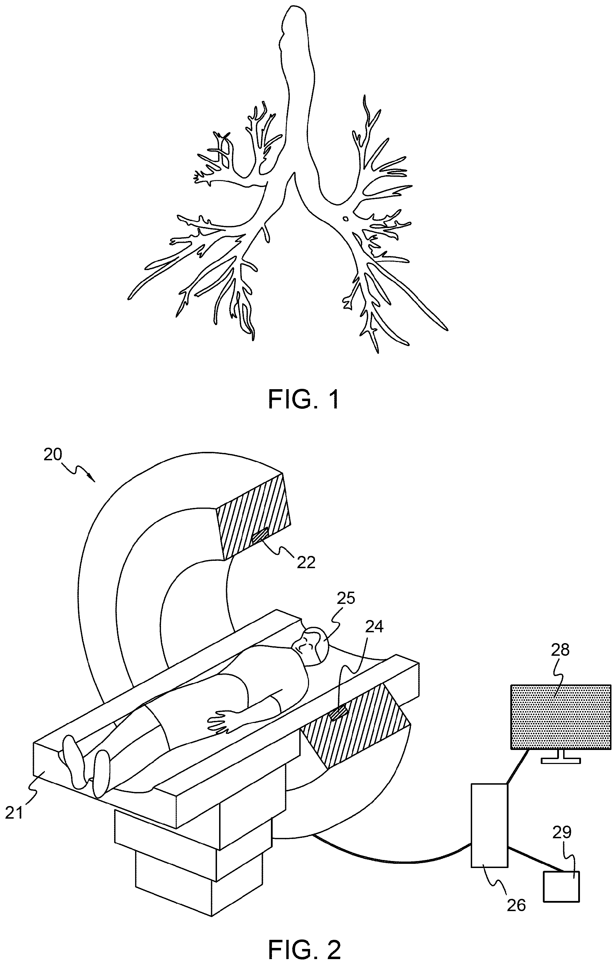

For non-invasive bronchial diagnostic and other medical procedures, it is desirable to provide an accurate bronchial airways representation of a subject's airways, i.e. the subject's bronchial tree. While the chambers of the heart are relatively large, the airways of the lungs form a tree, as illustrated in Fig. 1, with the trachea as the trunk from which bronchi extend as branches in primary, secondary, tertiary, etc. bronchi of diminishing sizes and tissue density as permeability to gas exchange increases.

As a result, the conventional use of CT scans to provide a three-dimensional representation of the airways of a subject's lungs commonly reflect inaccurate breaks or discontinuities of airways within a model derived from a CT scan. When such a representation is to be used for the navigation of a catheter or probe into distal regions of the airway tree, conventional airways representations show dead ends where in fact an airway continues in the subject.

Due to the complexity of the lungs, when a lung CT scan is segmented, sometimes a portion of the anatomy is missed. Further, when lung segmentations are inaccurate, the various airways may not appear to be connected or a physician may not be able to recognize where the airways are coming from, where they go, or how they are connected. The resulting anatomical model can be misleading to the physician. Thus, physicians can benefit from a refined approach to detect the entirety of most airways in a segmentation representation of a patient's bronchial airways.

Applicants have recognized that the use of the morphology of a corresponding representation of a subject's lungs' blood vessels can provide an enhanced airways representation. Blood vessels are often more accurately detected and represented than airways when a CT scan is conducted. Additionally, techniques such as introducing a contrast agent intravenously to a patient may be used to further enhance radiologic representation of vasculature. As discussed in more detail below, information regarding blood vessels can be used to extrapolate better information regarding unrepresented airways in the vicinity of the vessels to produce an enhanced representation of the subject's bronchial airways.

The inventors have recognized that vessels in the lungs are always accompanied by airways so that when a vessel is identified, there should always be a corresponding airway in its vicinity. Accordingly, by using the identification of corresponding vessels, missing parts of a segmentation representation of bronchial airways can be completed to provide an enhanced segmentation representation.

Fig. 2 is an illustration of an example computed tomography (CT) scan system 20 with a section removed for illustrative purposes. In this example, radiographic imaging is used to produce a sequence of two dimensional X-ray cross sections as a mobile bed or platform 21 moves a subject 25 through the scan system 20. Each radiographic cross section is a composite generated from multiple X-ray signals sent from multiple angles by a rapidly rotating X-ray generator or tube 22 opposite an X-ray detector 24 about the subject 25 that is on the mobile bed or platform 21 in a process called segmentation, with each individual cross section referred to as a segment. A processing unit 26 is provided that is configured to conduct digital geometry processing where data pertaining to the three dimensional volume of the scanned structure is determined along with the image data, and a coordinate map of the scan is thereby obtainable.

The sequence of segments derived from segmentation and its associated image and position coordinate data can be interpreted in several ways to provide an enhanced view of the subject's internal organs on a display 28 and/or saved to a memory 29 for use in medical diagnostics or other medical procedures. The three-dimensional aspect of a CT scan representation is generally derived from X, Y image coordinates relative to each cross section or segment with a Z spatial coordinate derived from the position of the given segment in the series of segments that make up the scan.

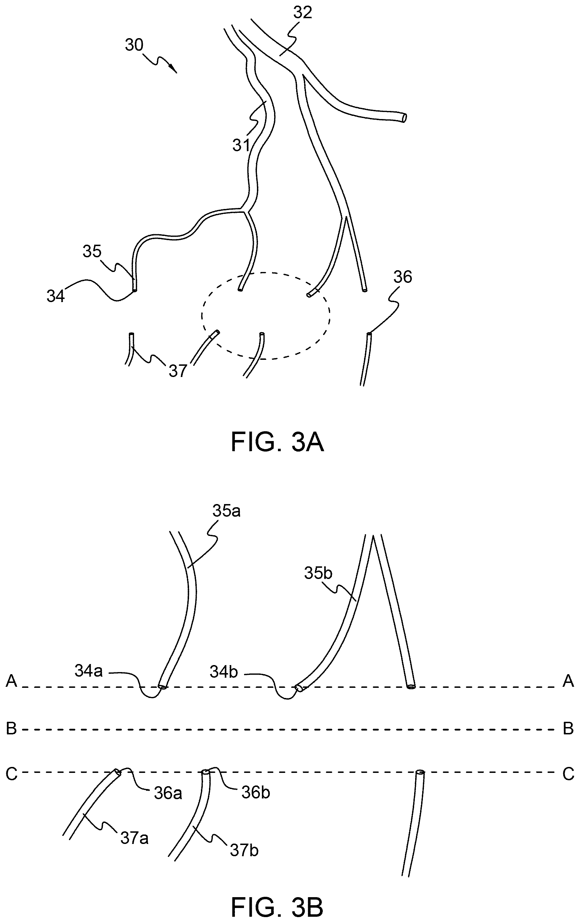

Fig. 3A is an example graphic illustration of a portion of a bronchial segmentation representation 30 of airways 31 branching from secondary bronchi 32 of the subject 25 who has had a CT scan. Convention methods for generating such a representation include setting a Hounsfield unit threshold for region growing a segmentation of the CT scan for airway detection. The example scan segmentation 30 reflects a proximal terminus 34 for each of several airways 35 and a distal terminus 36 for each of several disconnected airways 37; proximal and distal being relative to the undetected/unrepresented portions of the airways. Such discontinuities of airways are commonly found in conventional CT scan segmentation representations of lung airways since processing software is unable to represent some portions of airways which are unidentifiable in given segments of a CT scan. Typically, spatial coordinates of each airway terminus 34, 36 are identifiable from segment image data coordinates in association with the position of the given segment in the series of segments that make up the scan.

Fig. 3B provides an enlarged view of the circled portion of Fig. 3A where a terminus 34a is identified for an airway 35a, a terminus 34b is identified for an airway 35b, a terminus 36a is identified for an airway 37a and a terminus 36b is identified for an airway 37b.

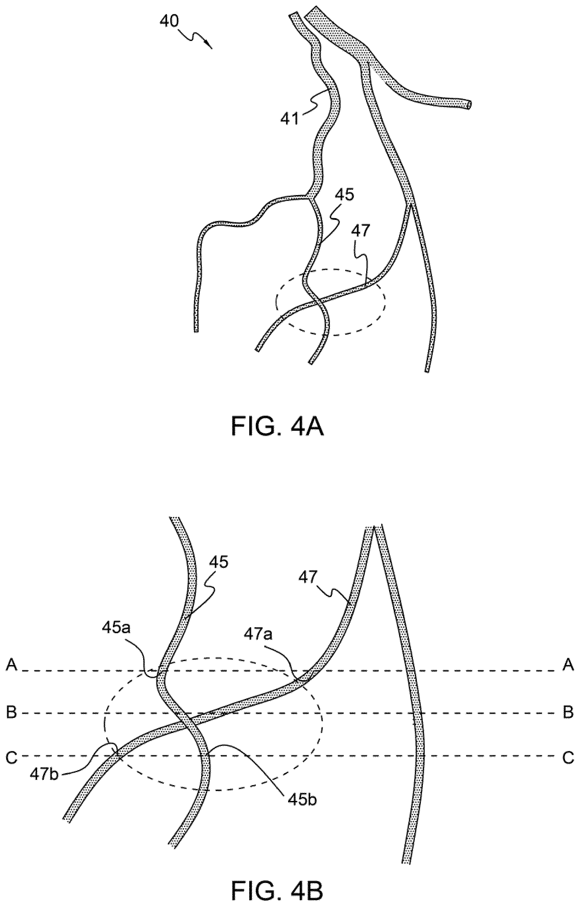

To provide an enhanced segmentation representation for airways segmentation 30, the morphology of a three dimensional representation of the vascular network that tracks the bronchial airway tree of the subject is utilized. Fig. 4A is an example graphic illustration of a portion of a vascular network segmentation representation 40 of the blood vessels 41 that correspond to the lung airways 31 of the subject 25 who has had a CT scan. Fig. 4B provides an enlarged view of the circled portion of Fig. 4A. The example vessel scan segmentation 40 reflects continuous vessels 45, 47 that span across an area corresponding to the discontinuous area between the proximal termini 34a, 34b, of the airways 35a, 35b and the distal termini 36a, 36b of the airways 37a, 37b shown in Fig 3B.

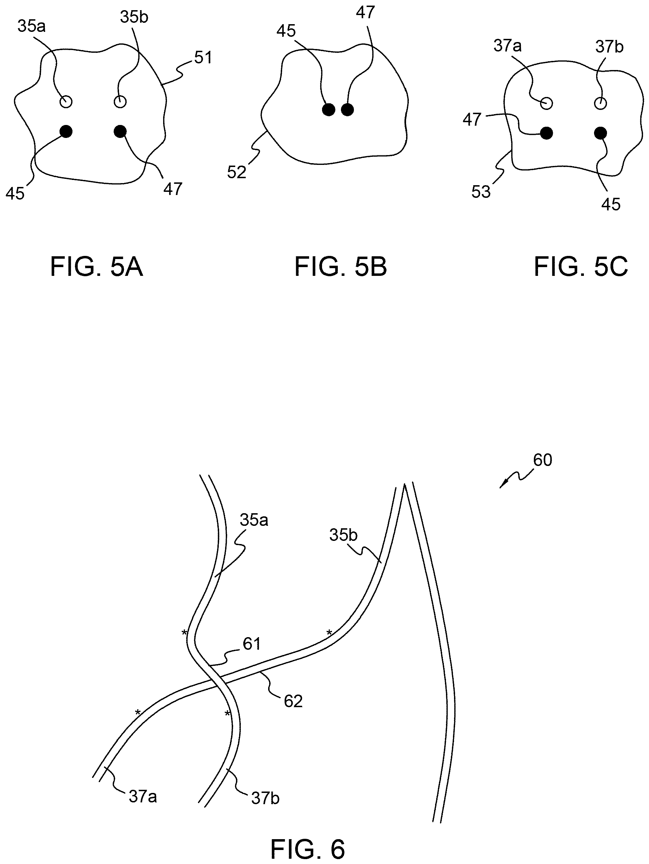

Figs. 5A-C are examples of portions of sequential two dimensional segments or cross sections from which the example airways segmentation 30 and vascular segmentation representation 40 were derived. Fig. 5A illustrates a portion of a segment 51 from which the portions for the airways and vascular segmentation representations 30, 40 at lines A-A of Figs 3B and 4B are derived. Fig. 5B illustrates a portion of a segment 52 from which the portions for the airways and vascular segmentation representations 30, 40 at lines B-B of Figs 3B and 4B are derived. Fig. 5C illustrates a portion of a segment 53 from which the portions for the airways and vascular segmentation representations 30, 40 at lines C-C of Figs 3B and 4B are derived.

From the segment 51 illustrated in Fig. 5A, portions of airways 35a and 35b of Fig. 3B are derived as well as portions of vessels 45 and 47 of Fig. 4B. From the segment 52 illustrated in Fig. 5B, airways cannot be ascertained which reflects the discontinuity between termini 34a, 34b and termini 36a, 36b of Fig. 3B. However, the continuity and pathway of the vessels 45 and 47 is able to be ascertained and represented as reflected in Fig. 4B. From the segment 53 illustrated in Fig. 5C, portions of airways 37a and 37b of Fig. 3B are derived as well as portions of vessels 45 and 47 of Fig. 4B, which when compared to the vessels 45 and 47 segment of Fig. 5A have reversed position due to the crossing of vascular pathways.

By correlating the spatial data of the airways segmentation 30 and vascular network representation 40, the airways segmentation 30 can be improved to provide an enhanced airways segmentation representation. As illustrated in Fig. 6, a portion of an enhanced airways segmentation representation 60 is shown that provides an improved version of the portion of the airways segmentation 30 shown in Fig. 3B. In the enhanced segmentation 60, airway 35a is continuous with airway 37b via enhanced airway portion 61 since that airway corresponds with the vessel 45. Similarly, airway 35b is continuous with airway 37a via enhanced airway portion 62 since that airway corresponds with the vessel 47. Asterisks (*) in Fig. 6 indicate the coordinate positions of termini 34a, 34b, 36a and 36b which have been eliminated as a result of the airway representation continuity via the enhanced airway portions 61 and 62.

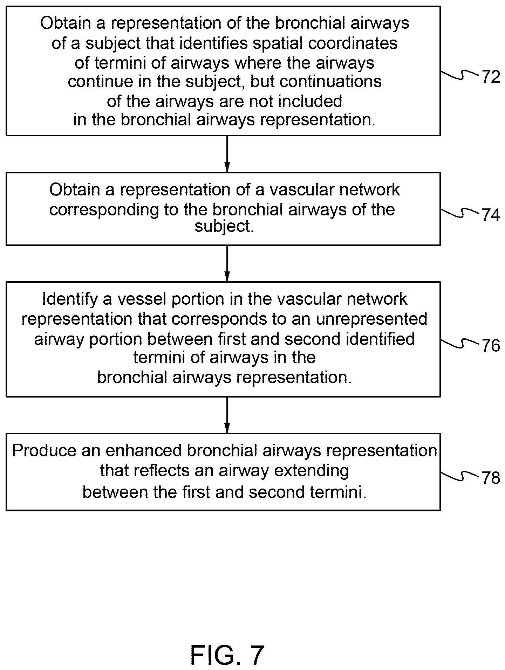

An example method for producing the enhanced representation of bronchial airways of a subject, such as enhanced segmentation 60, is provided with reference to Fig. 7. In an initial step 72, a representation of the bronchial airways of a subject is obtained that identifies spatial coordinates of termini of airways where the airways continue in the subject, but continuations of the airways are not included in the bronchial airways representation. For example, bronchial airways representation 30 can be obtained of the subject 25 using CT system 20.

A representation of a vascular network corresponding to the bronchial airways of the subject is also obtained, step 74. For example, vascular network representation 40 can be obtained of the subject 25 using CT system 20. However, it is not essential that the vascular representation be made at the same time or using the same method since the spatial coordinates of vessel branching can be coordinated with spatial coordinates of airway branching to provide sufficient correspondence data to perform the method. For example, the vascular representation can be obtained from a separate computed tomography scan that has been enhanced via a detectable substance such as, for example, a contrast agent.

In step 76, a vessel portion in the vascular network representation is identified that corresponds to an unrepresented airway portion between first and second identified termini of airways in the bronchial airways representation. To do this, locations in the vascular network representation can be identified that correspond to the identified termini of airways in the bronchial airways representation. For example, if segment 51 is the last segment where airways 35a and 35b can be ascertained, that segment can define the spatial location of the respective termini 34a, 34b. A search of the segment 51 can then be made for the closest blood vessel, in order, for example, to identify the vessel locations 45a, 47a, illustrated in Fig. 4B, as corresponding to the airway termini 34a, 34b, illustrated in Fig. 3B. This can be done through an automated search process of the data used to create the representations of airways 35a, 35b and vessels 45, 47 or by a physician or technician examining corresponding images of the vascular representation on a display to make the determination. To assure that the correct vessel locations are determined, consecutive segments may be examined to determine if the selected blood vessel actually corresponds to the airway in question.

Similarly, if segment 53 is the first segment where airways 37a and 37b can be ascertained, that segment can define the spatial location of the respective termini 36a, 36b. A search of the segment 53 can then be made for the closest blood vessel, in order, for example, to identify the vessel locations 45b, 47b, illustrated in Fig. 4B, as corresponding to the airway termini 36a, 36b, illustrated in Fig. 3B. This can be done through an automated search process of the data used to create the representations of airways 37a, 37b and vessels 45, 47 or by a physician or technician examining corresponding images to make the determination.

Upon a condition that two identified locations in the vascular network representation are connected by a vascular path of a blood vessel represented in the vascular network representation, the bronchial airways representation can be enhanced by indicating an airway extending between the two termini that correspond to the vascular path of the two identified locations. For example in Fig. 4B, the corresponding vascular locations 45a, 45b are connected by the blood vessel 45. Since vessel locations 45a, 45b correspond to termini 34a, 36b, termini 34a, 36b are then selected as the first and second termini that correspond to the proximal and distal termini of a portion of an airway that is unrepresented. Similarly, the corresponding vascular locations 47a, 47b are connected by the blood vessel 47. Since vessel locations 47a, 47b correspond to termini 34b, 36a, termini 34b, 36a can be selected as the first and second termini that correspond to the proximal and distal termini of a portion of an airway that is unrepresented.

In step 78, an enhanced bronchial airways representation is produced that reflects an airway extending between the first and second termini that have accordingly been determined as the ends of the unrepresented airway. For example, in the enhanced airways representation 60, airway portion 61 is provided between airways 35a, 37b. Similarly, an airway portion 62 is provided between airways 35b, 37a. This can be accomplished, for example, by setting a lower Hounsfield unit threshold for region growing the segmentation from the CT scan around detected vessels both in terms of location and orientation of detected vessels.

In one example, a processor and associated memory, for example the processor 26 and the associated memory 29 of Fig. 1, can be configured to provide an enhance representation of a CT-scan of bronchial airways of a subject that can be displayed on a monitor, such as the monitor 28.

The memory 29 can be configured to store a data representation of the bronchial airways of the subject 25 that identifies spatial coordinates of termini of airways where the airways continue in the subject, but continuations of the airways are not included in the bronchial airways representation. The memory can also be configured to store a data representation of a vascular network corresponding to the bronchial airways of the subject.

The processor 26 can be configured to identify a vessel portion in the vascular network representation that corresponds to an unrepresented airway portion between first and second identified termini of airways in the bronchial airways representation. The processor 26 can then be configured to produce an enhanced bronchial airways representation that reflects an airway extending between the first and second termini that can be displayed on the display 28.

To do this the processor 26 can be configured to identify locations in the vascular network representation that correspond to the identified termini of airways in the bronchial airways representation, such as corresponding vascular locations 45a, 45b, 47a and 47b in Fig. 4B. The processor can also be configured to determine when two identified locations in the vascular network representation are connected by a vascular path of a blood vessel represented in the vascular network representation to thereby enable enhancement of the bronchial airways representation by indicating an airway extending between the two termini that correspond to the vascular path of the two identified locations.

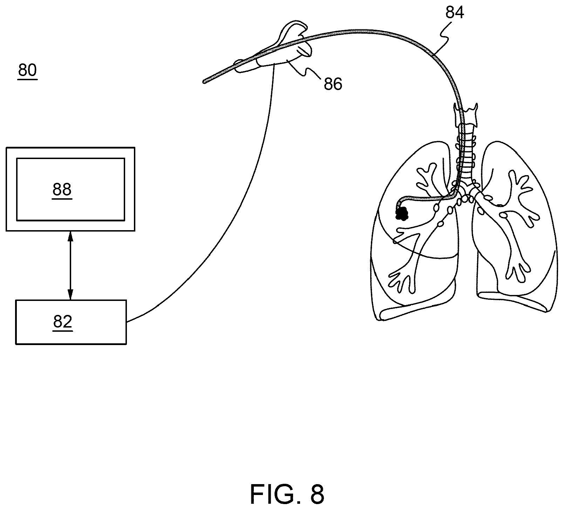

The bronchial airways representation enhancement system is not dependent on being incorporated into a CT-Scan system such as the system 20 depicted in Fig. 2. The bronchial airways representation enhancement system can advantageously be incorporated into a medical apparatus used to perform diagnostic and other bronchoscopic procedures such as the medical apparatus 80 depicted in Fig. 8.

The example medical apparatus 80 includes a controller 82 that controls the positioning of a bronchoscope 84 via a robotic handle 86 for conducting a diagnostic or other bronchoscopic procedure. Navigation of a distal end of the bronchoscope 84 is facilitated by displaying a representation of the airways of a patient on a system monitor 88.

The controller 82 includes processor and memory components for facilitating the operation of the medical apparatus 80. In such a system 80, data for previously generated bronchial airways and vascular network representations can be stored in memory of the controller 82 which can be configured to display those representations on the system monitor 88. The controller 82 can be configured to process the previously generated bronchial airways representation referencing the morphology of the previously generated vascular network representation to produce and display an enhanced bronchial airways representation as discussed above. This is highly advantageous when it is desired to navigate the bronchoscope 86 to a position in the patient's lungs that must traverse portions of airways that are not represented in the previously generated bronchial airways representation.

The examples discussed above are non-limiting. Although representations based on CT-scan are used for the above examples, the invention is applicable to representations derived from use of other technologies as well. Other variations and embodiments will be apparent to those skilled in the art within the scope of the appended claims.

## Claims

1. A method for providing an enhanced representation of bronchial airways (31, 35, 35a, 35b, 37a, 37b) of a subject comprising:
obtaining a representation of the bronchial airways of a subject that identifies spatial coordinates of termini of airways (34, 34a, 34b, 36, 36a, 36b) where the airways continue in the subject, but continuations of the airways are not included in the bronchial airways representation;
obtaining a representation of a vascular network (40) corresponding to the bronchial airways of the subject;
identifying a vessel portion (45, 47) in the vascular network representation that corresponds to an unrepresented airway portion between first and second identified termini of airways in the bronchial airways representation; and
producing an enhanced bronchial airways representation (60) that reflects an airway extending between the first and second termini.

2. The method according to claim 1 wherein the identifying a vessel portion in the vascular network representation that corresponds to an unrepresented airway between first and second identified termini of airways in the bronchial airways representation includes:
identifying locations in the vascular network representation that correspond to the identified termini of airways in the bronchial airways representation; and
upon a condition that two identified locations in the vascular network representation are connected by a vascular path of a blood vessel represented in the vascular network representation, selecting the identified termini as the first and second termini for which the enhanced bronchial airways representation reflects an airway extending therebetween.

3. The method according to claim 2 wherein the enhanced bronchial airways representation is displayed on a display.

4. The method according to claim 3 wherein the displayed enhanced bronchial airways representation is used to assist navigation in a bronchoscopic procedure.

5. The method according to claim 1 wherein the bronchial airways representation and the vascular network representation are obtained from a single computed tomography CT scan wherein the bronchial airways representation was generated by setting a Hounsfield unit threshold for region growing segmentation of the CT scan for airway detection and the enhanced bronchial airways representation is generated by setting a lower Hounsfield unit threshold for region growing segmentation on the CT scan around detected vessels both in terms of location and orientation of detected vessels.

6. The method according to claim 1 wherein the bronchial airways representation is obtained from a computed tomography scan and the vascular network representation is obtained from a separate computed tomography scan.

7. The method according to claim 6 wherein the vascular network representation is obtained from a computed tomography scan that has been enhanced via a contrast agent.

8. The method according to claim 2 wherein a display of the vascular network representation is used in identifying a vessel portion in the vascular network representation that corresponds to an unrepresented airway portion to enable the identifying of locations to be conducted manually.

9. The method according to claim 2 wherein the identifying of locations in the vascular network representation that correspond to the termini of airways in the bronchial airways is determined by a processor.

10. The method according to claim 1 further comprising displaying the enhanced bronchial airways representation on a monitor of a medical apparatus to facilitate a bronchoscopic procedure.

11. An apparatus for providing an enhanced representation of bronchial airways (31, 35, 35a, 35b, 37a, 37b) of a subject comprising:
a memory configured to store a data representation of the bronchial airways of a subject that identifies spatial coordinates of termini of airways (34, 34a, 34b, 36, 36a, 36b) where the airways continue in the subject, but continuations of the airways are not included in the bronchial airways representation;
the memory configured to store a data representation of a vascular network (40) corresponding to the bronchial airways of the subject;
a processor configured to identify a vessel portion (45, 47) in the vascular network representation that corresponds to an unrepresented airway portion between first and second identified termini of airways in the bronchial airways representation; and
the processor configured to produce an enhanced bronchial airways representation (60) that reflects an airway extending between the first and second termini.

12. The apparatus according to claim 11 wherein the processor is configured to identify a vessel portion in the vascular network representation that corresponds to an unrepresented airway between first and second identified termini of airways in the bronchial airways representation by:
identifying locations in the vascular network representation that correspond to the identified termini of airways in the bronchial airways representation; and
upon a condition that two identified locations in the vascular network representation are connected by a vascular path of a blood vessel represented in the vascular network representation, selecting the two identified termini as the first and second termini for which the enhanced bronchial airways representation reflects an airway extending therebetween.

13. The apparatus according to claim 12 further comprising a display configured to display the enhanced bronchial airways representation.

14. The apparatus according to claim 13 wherein display is a monitor of a medical apparatus configured to assist navigation of a bronchoscope in a bronchoscopic procedure.

15. The apparatus according to claim 11 wherein:
the memory is configured to store the bronchial airways representation and the vascular network representation that were obtained from a single computed tomography scan wherein the bronchial airways representation was generated by setting a Hounsfield unit threshold for region growing segmentation of the CT scan for airway detection; and
the processor is configured to generate the enhanced bronchial airways representation by setting a lower Hounsfield unit threshold for region growing segmentation on the CT scan around detected vessels both in terms of location and orientation of detected vessels.

16. The apparatus according to claim 11 wherein the memory is configured to store the bronchial airways representation as obtained from a computed tomography scan and the memory is configured to store the vascular network representation as obtained from a separate computed tomography scan.

17. The apparatus according to claim 16 wherein the memory is configured to store the vascular network representation as obtained from a computed tomography scan that has been enhanced via a contrast agent.

18. The apparatus according to claim 12 further comprising a display configured to display the vascular network representation that is used in identifying a vessel portion in the vascular network representation that corresponds to an unrepresented airway portion to enable the location identification to be conducted manually.

19. The apparatus according to claim 12 wherein the processor is configured to identify locations in the vascular network representation that correspond to the termini of airways in the bronchial airways representation.

20. The apparatus according to claim 11 further comprising a display configured to display the enhanced bronchial airways representation that is part of a medical apparatus configured to facilitate a bronchoscopic procedure.

## Patentansprüche

1. Verfahren zur Bereitstellung einer verbesserten Darstellung der bronchialen Atemwege (31, 35, 35a, 35b, 37a, 37b) eines Individuums, umfassend:
Erhalten einer Darstellung der bronchialen Atemwege eines Individuums, die räumliche Koordinaten von Termini der Atemwege (34, 34a, 34b, 36, 36a, 36b) identifiziert, wo sich die Atemwege im Individuum fortsetzen, wobei aber Fortsetzungen der Atemwege in der Darstellung der bronchialen Atemwege nicht enthalten sind;
Erhalten einer Darstellung eines Gefäßnetzes (40), das den bronchialen Atemwegen des Individuums entspricht;
Identifizieren eines Gefäßabschnitts (45, 47) in der Gefäßnetzdarstellung, der einem nicht dargestellten Atemwegsabschnitt zwischen ersten und zweiten identifizierten Termini der Atemwege in der Darstellung der bronchialen Atemwege entspricht; und
Erstellen einer verbesserten Darstellung (60) der bronchialen Atemwege, die einen Atemweg widerspiegelt, der sich zwischen ersten und zweiten Termini erstreckt.

2. Verfahren nach Anspruch 1, wobei das Identifizieren eines Gefäßabschnitts in der Gefäßnetzdarstellung, der einem nicht dargestellten Atemweg zwischen ersten und zweiten identifizierten Termini der Atemwege in der Darstellung der bronchialen Atemwege entspricht, das Folgende beinhaltet:
Identifizieren von Stellen in der Gefäßnetzdarstellung, die den identifizierten Termini von Atemwegen in der Darstellung der bronchialen Atemwege entsprechen; und
unter einer Bedingung, dass zwei identifizierte Stellen in der Gefäßnetzdarstellung durch einen Gefäßpfad eines Blutgefäßes, das in der Gefäßnetzdarstellung dargestellt wird, miteinander verbunden sind, Auswählen der identifizierten Termini als erste und zweite Termini, für die die verbesserte Darstellung der bronchialen Atemwege einen sich dazwischen erstreckenden Atemweg widerspiegelt.

3. Verfahren nach Anspruch 2, wobei die verbesserte Darstellung der bronchialen Atemwege auf einer Anzeige angezeigt wird.

4. Verfahren nach Anspruch 3, wobei die angezeigte verbesserte Darstellung der bronchialen Atemwege zur Unterstützung der Navigation in einem bronchoskopischen Verfahren benutzt wird.

5. Verfahren nach Anspruch 1, wobei die Darstellung der bronchialen Atemwege und die Gefäßnetzdarstellung aus einer einzelnen Computertomographie-(CT)-Aufnahme erhalten werden, wobei die Darstellung der bronchialen Atemwege durch Einstellen eines Schwellenwerts für die Hounsfield-Einheit zur Region-Growing-Segmentierung der CT-Aufnahme für den Nachweis von Atemwegen erzeugt wurde und die verbesserte Darstellung der bronchialen Atemwege durch Einstellen eines niedrigeren Schwellenwerts für die Hounsfield-Einheit zur Region-Growing-Segmentierung auf der CT-Aufnahme um nachgewiesene Gefäße, sowohl im Hinblick auf Lokalisation als auch auf Orientierung nachgewiesener Gefäße, erzeugt wird.

6. Verfahren nach Anspruch 1, wobei die Darstellung der bronchialen Atemwege aus einer Computertomographieaufnahme erhalten wird und die Gefäßnetzdarstellung aus einer gesonderten Computertomographieaufnahme erhalten wird.

7. Verfahren nach Anspruch 6, wobei die Gefäßnetzdarstellung aus einer Computertomographieaufnahme erhalten wird, die mit einem Kontrastmittel verstärkt wurde.

8. Verfahren nach Anspruch 2, wobei eine Anzeige der Gefäßnetzdarstellung bei der Identifizierung eines Gefäßabschnitts in der Gefäßnetzdarstellung, der einem nicht dargestellten Atemwegsabschnitt entspricht, verwendet wird, um die manuelle Durchführung der Identifizierung von Stellen zu ermöglichen.

9. Verfahren nach Anspruch 2, wobei die Identifizierung von Stellen in der Gefäßnetzdarstellung, die den Termini von Atemwegen in den bronchialen Atemwegen entsprechen, von einem Prozessor bestimmt wird.

10. Verfahren nach Anspruch 1, ferner umfassend die Anzeige der verbesserten Darstellung der bronchialen Atemwege auf einem Bildschirm einer medizinischen Vorrichtung zur Erleichterung eines bronchoskopischen Verfahrens.

11. Vorrichtung zur Bereitstellung einer verbesserten Darstellung der bronchialen Atemwege (31, 35, 35a, 35b, 37a, 37b) eines Individuums, umfassend:
einen Speicher, der zum Speichern einer Datendarstellung der bronchialen Atemwege eines Individuums, die räumliche Koordinaten von Termini von Atemwegen (34, 34a, 34b, 36, 36a, 36b) identifiziert, wo sich die Atemwege im Individuum fortsetzen, wobei aber Fortsetzungen der Atemwege in der Darstellung der bronchialen Atemwege nicht enthalten sind, ausgelegt ist;
wobei der Speicher zum Speichern einer Datendarstellung eines Gefäßnetzes (40), das den bronchialen Atemwegen des Individuums entspricht, ausgelegt ist;
einen Prozessor, der zum Identifizieren eines Gefäßabschnitts (45, 47) in der Gefäßnetzdarstellung, der einem nicht dargestellten Atemwegsabschnitt zwischen ersten und zweiten identifizierten Termini der Atemwege in der Darstellung der bronchialen Atemwege entspricht, ausgelegt ist; und
wobei der Prozessor zum Erstellen einer verbesserten Darstellung (60) der bronchialen Atemwege, die einen Atemweg widerspiegelt, der sich zwischen ersten und zweiten Termini erstreckt, ausgelegt ist.

12. Vorrichtung nach Anspruch 11, wobei der Prozessor zum Identifizieren eines Gefäßabschnitts in der Gefäßnetzdarstellung, der einem nicht dargestellten Atemweg zwischen ersten und zweiten identifizierten Termini der Atemwege in der Darstellung der bronchialen Atemwege entspricht, ausgelegt ist, durch:
Identifizieren von Stellen in der Gefäßnetzdarstellung, die den identifizierten Termini von Atemwegen in der Darstellung der bronchialen Atemwege entsprechen; und
unter einer Bedingung, dass zwei identifizierte Stellen in der Gefäßnetzdarstellung durch einen Gefäßpfad eines Blutgefäßes, das in der Gefäßnetzdarstellung dargestellt wird, miteinander verbunden sind, Auswählen der zwei identifizierten Termini als erste und zweite Termini, für die die verbesserte Darstellung der bronchialen Atemwege einen sich dazwischen erstreckenden Atemweg widerspiegelt.

13. Vorrichtung nach Anspruch 12, ferner umfassend eine Anzeige, die zum Anzeigen der verbesserten Darstellung der bronchialen Atemwege ausgelegt ist.

14. Vorrichtung nach Anspruch 13, wobei die Anzeige ein Bildschirm einer medizinischen Vorrichtung ist, die zur Unterstützung der Navigation eines Bronchoskops in einem bronchoskopischen Verfahren ausgelegt ist.

15. Vorrichtung nach Anspruch 11, wobei:
der Speicher zum Speichern der Darstellung der bronchialen Atemwege und der Gefäßnetzdarstellung ausgelegt ist, die aus einer einzelnen Computertomographieaufnahme erhalten wurden, wobei die Darstellung der bronchialen Atemwege durch Einstellen eines Schwellenwerts für die Hounsfield-Einheit zur Region-Growing-Segmentierung der CT-Aufnahme für den Nachweis von Atemwegen erzeugt wurde; und
der Prozessor zum Erzeugen der verbesserten Darstellung der bronchialen Atemwege durch Einstellen eines niedrigeren Schwellenwerts für die Hounsfield-Einheit zur Region-Growing-Segmentierung auf der CT-Aufnahme um nachgewiesene Gefäße, sowohl im Hinblick auf Lokalisation als auch auf Orientierung nachgewiesener Gefäße, ausgelegt ist.

16. Vorrichtung nach Anspruch 11, wobei der Speicher zum Speichern der aus einer Computertomographieaufnahme erhaltenen Darstellung der bronchialen Atemwege ausgelegt ist und der Speicher zum Speichern der aus einer gesonderten Computertomographieaufnahme erhaltenen Gefäßnetzdarstellung ausgelegt ist.

17. Vorrichtung nach Anspruch 16, wobei der Speicher zum Speichern der aus einer Computertomographieaufnahme, die mit einem Kontrastmittel verstärkt wurde, erhaltenen Gefäßnetzdarstellung ausgelegt ist.

18. Vorrichtung nach Anspruch 12, ferner umfassend eine Anzeige, die zum Anzeigen der Gefäßnetzdarstellung, die bei der Identifizierung eines Gefäßabschnitts in der Gefäßnetzdarstellung, der einem nicht dargestellten Atemwegsabschnitt entspricht, verwendet wird, um die manuelle Durchführung der Identifizierung von Stellen zu ermöglichen, ausgelegt ist.

19. Vorrichtung nach Anspruch 12, wobei der Prozessor zum Identifizieren von Stellen in der Gefäßnetzdarstellung, die den Termini von Atemwegen in der Darstellung der bronchialen Atemwege entsprechen, ausgelegt ist.

20. Vorrichtung nach Anspruch 11, ferner umfassend eine Anzeige, die zum Anzeigen der verbesserten Darstellung der bronchialen Atemwege ausgelegt ist, die Teil einer medizinischen Vorrichtung ist, die zur Erleichterung eines bronchoskopischen Verfahrens ausgelegt ist.

## Revendications

1. Procédé permettant de fournir une représentation améliorée de bronches (31, 35, 35a, 35b, 37a, 37b) d'un sujet, consistant à :
obtenir une représentation des bronches d'un sujet qui identifie les coordonnées spatiales des terminaisons des voies respiratoires (34, 34a, 34b, 36, 36a, 36b) où les voies respiratoires se prolongent dans le sujet, mais les prolongements des voies respiratoires ne sont pas inclus dans la représentation des bronches ;
obtenir une représentation d'un réseau vasculaire (40) correspondant aux bronches du sujet ;
identifier une partie de vaisseau (45, 47) dans la représentation du réseau vasculaire qui correspond à une partie des voies respiratoires non représentée entre les première et seconde terminaisons identifiées des voies respiratoires dans la représentation des bronches ; et
produire une représentation améliorée des bronches (60) qui reflète une voie respiratoire s'étendant entre les première et seconde terminaisons.

2. Procédé selon la revendication 1, dans lequel l'identification d'une partie de vaisseau dans la représentation du réseau vasculaire qui correspond à une voie respiratoire non représentée entre les première et seconde terminaisons identifiées des voies respiratoires dans la représentation des bronches consiste à :
identifier des emplacements dans la représentation du réseau vasculaire qui correspondent aux terminaisons identifiées des voies respiratoires dans la représentation des bronches ; et
à condition que deux emplacements identifiés dans la représentation du réseau vasculaire soient reliés par une voie vasculaire d'un vaisseau sanguin représenté dans la représentation du réseau vasculaire, sélectionner les terminaisons identifiées comme étant les première et seconde terminaisons pour lesquelles la représentation améliorée des bronches reflète une voie respiratoire s'étendant entre celles-ci.

3. Procédé selon la revendication 2, dans lequel la représentation améliorée des bronches est affichée sur un écran.

4. Procédé selon la revendication 3, dans lequel la représentation améliorée des bronches est utilisée pour aider à la navigation dans une intervention bronchoscopique.

5. Procédé selon la revendication 1, dans lequel la représentation des bronches et la représentation du réseau vasculaire sont obtenues à partir d'un seul tomodensitogramme dans lequel la représentation des bronches a été générée en définissant un seuil d'unité Hounsfield pour une segmentation en régions du tomodensitogramme pour la détection des voies respiratoires et la représentation améliorée des bronches est générée en définissant un seuil d'unité Hounsfield inférieur pour la segmentation en régions sur le tomodensitogramme autour des vaisseaux détectés à la fois en termes d'emplacements et d'orientation des vaisseaux détectés.

6. Procédé selon la revendication 1, dans lequel la représentation des bronches est obtenue à partir d'un tomodensitogramme et la représentation du réseau vasculaire est obtenue à partir d'un tomodensitogramme distinct.

7. Procédé selon la revendication 6, dans lequel la représentation du réseau vasculaire est obtenue à partir d'un tomodensitogramme qui a été amélioré par le biais d'un agent de contraste.

8. Procédé selon la revendication 2, dans lequel un affichage de la représentation du réseau vasculaire est utilisé dans l'identification d'une partie de vaisseau dans la représentation du réseau vasculaire qui correspond à une partie des voies respiratoires non représentée pour permettre l'identification manuelle des emplacements.

9. Procédé selon la revendication 2, dans lequel l'identification d'emplacements dans la représentation du réseau vasculaire qui correspondent aux terminaisons des voies respiratoires dans les bronches est déterminée par un processeur.

10. Procédé selon la revendication 1, consistant en outre à afficher la représentation améliorée des bronches sur un moniteur d'un appareil médical pour faciliter une intervention bronchoscopique.

11. Appareil permettant de fournir une représentation améliorée de bronches (31, 35, 35a, 35b, 37a, 37b) d'un sujet comprenant :
une mémoire configurée pour mémoriser une représentation de données des bronches d'un sujet qui identifie les coordonnées spatiales des terminaisons des voies respiratoires (34, 34a, 34b, 36, 36a, 36b) où les voies respiratoires se prolongent dans le sujet, mais les prolongements des voies respiratoires ne sont pas inclus dans la représentation des bronches ;
la mémoire configurée pour mémoriser une représentation de données d'un réseau vasculaire (40) correspondant aux bronches du sujet ;
un processeur configuré pour identifier une partie de vaisseau (45, 47) dans la représentation du réseau vasculaire qui correspond à une partie des voies respiratoires non représentée entre les première et seconde terminaisons identifiées des voies respiratoires dans la représentation des bronches ; et
le processeur configuré pour produire une représentation améliorée des bronches (60) qui reflète une voie respiratoire s'étendant entre les première et seconde terminaisons.

12. Appareil selon la revendication 11, dans lequel le processeur est configuré pour identifier une partie de vaisseau dans la représentation du réseau vasculaire qui correspond à une voie respiratoire non représentée entre les première et seconde terminaisons identifiées des voies respiratoires dans la représentation des bronches par :
l'identification des emplacements dans la représentation du réseau vasculaire qui correspondent aux terminaisons identifiées des voies respiratoires dans la représentation des bronches ; et
à condition que deux emplacements identifiés dans la représentation du réseau vasculaire soient reliés par une voie vasculaire d'un vaisseau sanguin représenté dans la représentation du réseau vasculaire, la sélection des deux terminaisons identifiées comme étant les première et seconde terminaisons pour lesquelles la représentation améliorée des bronches reflète une voie respiratoire s'étendant entre celles-ci.

13. Appareil selon la revendication 12, comprenant en outre un affichage configuré pour afficher la représentation améliorée des bronches.

14. Appareil selon la revendication 13, dans lequel l'écran est un moniteur d'un appareil médical configuré pour aider à la navigation d'un bronchoscope dans une intervention bronchoscopique.

15. Appareil selon la revendication 11, dans lequel :
la mémoire est configurée pour mémoriser la représentation des bronches et la représentation du réseau vasculaire qui ont été obtenues à partir d'un seul tomodensitogramme dans lequel la représentation des bronches a été générée en définissant un seuil d'unité Hounsfield pour la segmentation en régions du tomodensitogramme pour la détection des voies respiratoires ; et
le processeur est configuré pour générer la représentation améliorée des bronches en définissant un seuil d'unité Hounsfield inférieur pour la segmentation en régions sur le tomodensitogramme autour des voies respiratoires détectées à la fois en termes d'emplacements et d'orientation des vaisseaux détectés.

16. Appareil selon la revendication 11, dans lequel la mémoire est configurée pour mémoriser la représentation des bronches obtenue à partir d'un tomodensitogramme et la mémoire est configurée pour mémoriser la représentation du réseau vasculaire obtenue à partir d'un tomodensitogramme distinct.

17. Appareil selon la revendication 16, dans lequel la mémoire est configurée pour mémoriser la représentation du réseau vasculaire obtenue à partir d'un tomodensitogramme qui a été amélioré par le biais d'un agent de contraste.

18. Appareil selon la revendication 12, comprenant en outre un affichage configuré pour afficher la représentation du réseau vasculaire qui est utilisée pour identifier une partie de vaisseau dans la représentation du réseau vasculaire qui correspond à une partie des voies respiratoires non représentée pour permettre l'identification manuelle des emplacements.

19. Appareil selon la revendication 12, dans lequel le processeur est configuré pour identifier des emplacements dans la représentation du réseau vasculaire qui correspondent aux terminaisons des voies respiratoires dans la représentation des bronches.

20. Appareil selon la revendication 11, comprenant en outre un écran configuré pour afficher la représentation améliorée des bronches qui fait partie d'un appareil médical configuré pour faciliter une intervention bronchoscopique.
